# FASCICULE DE BREVET EUROPEEN

(11) **EP 1 905 775 B1**
(45) Date de publication et mention de la délivrance du brevet: **08.09.2010**
(21) Numéro de dépôt: 07291135.7
(22) Date de dépôt: 25.09.2007
(51) Int. Cl.: C07D 498/04, A61K 31/538, A61P 25/00

(54) **Composés tétracycliques, leur procédé de préparation, les compositions pharmaceutiques qui les contiennent et leur utilisation comme ligands des récepteurs dopaminergiques.**
Tetrazykline, ihr Herstellungsverfahren, deren pharmazeutischen Zusammensetzungen, die sie enthalten und deren Verwendung als Dopamin-Rezeptoren Liganden
Tetracyclic compounds, method of preparing them, pharmaceutical compositions containing them and their use as dopamine receptors ligands

(30) Priorité: 26.09.2006 FR 0608413
(43) Date de publication de la demande: 02.04.2008
(73) Titulaire: Les Laboratoires Servier, 92415 Courbevoie Cedex (FR)
(72) Inventeur: Peglion, Jean-Louis, 78110 Le Vesinet (FR); Poitevin, Christophe, 75011 Paris (FR); Millan, Mark, 78230 Le Pecq (FR); Brocco, Mauricette, 75003 Paris (FR)
(74) Mandataire: Giudicelli, Cathy

(56) Documents cités:
- EP-A1- 0 686 637
- EP-A1- 0 773 223
- EP-A1- 0 899 267
- EP-A2- 0 080 115
- PEGLION, JEAN-LOUIS ET AL: "Tetracyclic analogs of [+]-S 14297: synthesis and determination of affinity and selectivity at cloned human dopamine D3 vs D2 receptors" BIOORGANIC & MEDICINAL CHEMISTRY LETTERS , 7(7), 881-886 CODEN: BMCLE8; ISSN: 0960-894X, 1997, XP004136149

## Description

La présente invention a pour objet de nouveaux composés tétracycliques, leur procédé de préparation et les compositions pharmaceutiques qui les contiennent.

EP 0 773 223 et EP 0 686 637 décrivent des composés tétracycliques agissant comme ligands dopaminergiques présentant une sélectivité envers les récepteurs D₃.

La présente invention concerne plus spécialement les composés de formule I, de configuration relative trans : 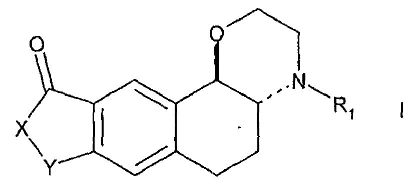 dans laquelle :
◆ X représente un atome d'oxygène ou un groupement NR₂,
◆ Y représente un groupement choisi parmi -CH₂-, -(CH₂)₂- et -CH=CH-,
◆ R₁ et R₂, identiques ou différents, représentent chacun un atome d'hydrogène ou un groupement choisi parmi alkyle C₁-C₆ linéaire ou ramifié, cycloalkyle C₃-C₈ et cycloalkylalkyle, dans lequel la partie alkyle est en C₁-C₆ et est linéaire ou ramifiée, et la partie cycloalkyle est en C₃-C₈,
sous forme racémique ou d'isomères optiques,
ainsi que leurs sels d'addition avec un acide pharmaceutiquement acceptable et leurs hydrates.

Par groupement cycloalkyle C₃-C₈, on entend un groupement hydrocarboné monocyclique saturé de 3 à 8 chaînons.

Parmi les acides pharmaceutiquement acceptables, on peut citer à titre non limitatif les acides chlorhydrique, bromhydrique, sulfurique, phosphorique, acétique, trifluoroacétique, lactique, pyruvique, malonique, succinique, glutarique, fumarique, tartrique, maléique, citrique, ascorbique, oxalique, méthanesulfonique, benzènesulfonique, camphorique, dibenzoyltartrique.

Un aspect de la présente invention concerne les composés de formule I pour lesquels R₁ représente un groupement alkyle, en particulier un groupement propyle.

Un autre aspect de la présente invention concerne les composés de formule I pour lesquels X représente un groupement NR₂, en particulier un groupement NH.

Un autre aspect de la présente invention concerne les composés de formule I pour lesquels Y représente un groupement CH₂.

Un autre aspect de la présente invention concerne les composés de formule I suivants :
- la (4a*RS* ,11b*RS*) 4-propyl-3,4,4a,5,6,8,9,11b-octahydroisoindolo[5,6-*h*][1,4]benzoxazin-10(2*H*)-one, ainsi que ses énantiomères et ses sels d'addition à un acide pharmaceutiquement acceptable;
- la (4a*R*,11b*R*) 4-propyl-2,3,4,4a,5,6,8,11b-octahydro-10*H*-furo[3',4':6,7] naphto[1,2-b][1,4] oxazin-10-one, ainsi que ses sels d'addition à un acide pharmaceutiquement acceptable;
- la (4a*R*,12b*R*) 4-propyl-3,4,4a,5,6,8,9,12b-octahydro-2*H*,11*H*-pyrano[4',3':6,7] naphto [1,2-b][1,4] oxazin-11-one, ainsi que ses sels d'addition à un acide pharmaceutiquement acceptable;
- la (4a*R,* 12b*R*) 4-propyl-2,3,4,4a,5,6,8,9,10,12b-décahydro-11*H* isoquino [6,7-*h*][1,4] benzoxazin-11-one, ainsi que ses sels d'addition à un acide pharmaceutiquement acceptable; et
- la (4a*R*, 12b*R*) 4-propyl-2,3,4,4a,5,6,10,12b-octahydro-11*H*- isoquino [6,7-*h*][1,4] benzoxazin-11-one, ainsi que ses sels d'addition à un acide pharmaceutiquement acceptable.

Les composés de formule I agissent comme de puissants ligands dopaminergiques.

Les dérivés dopaminergiques sont largement utilisés en thérapeutique grâce à leurs effets bénéfiques dans les maladies psychiatriques et neurologiques et en périphérie dans les maladies cardiovasculaires.

Cinq sous-types réceptoriels dopaminergiques (D₁ à D₅) ont été actuellement clonés et caractérisés. Actuellement, la grande majorité des médicaments de cette classe agissent au niveau du système dopaminergique par l'intermédiaire de leur action sur le sous-type D₂, qu'il s'agisse de bloqueurs (ou antagonistes) ou d'activateurs (ou agonistes). Ces médicaments présentent de nombreux effets secondaires : dyskinésie, hyperprolactinémie, aménorrhée pour les premiers, effets cardio-vasculaires et émétisants pour les seconds.

Contrairement aux récepteurs D₂, la concentration des récepteurs D₃ est très faible dans le noyau nigrostrié et dans les cellules lactotrophes (Pharmacol Ther. 2001, 90(2-3), 231-59; CNS Neurol Disord Drug Targets 2006, 5(1), 25-43). Par contre, à l'instar des récepteurs D₂, la concentration des récepteurs D₃ est très importante au niveau du système limbique *(*Pharmacol Ther. 2001, 90(2-3), 231-59; CNS Neurol Disord Drug Targets 2006, 5(1), 25-43). Cette différence importante dans la localisation de ces deux sous-types réceptoriels encourage la recherche de nouveaux médicaments agissant de façon préférentielle sur le sous-type D₃ et qui devrait se traduire par une minimisation des effets secondaires liés typiquement au sous-type D₂, comme mentionné précédemment *(*Pharmacol Ther. 2001, 90(2-3), 231-59; J Pharmacol Exp Ther. 2004, 309(3), 936-50; J Pharmacol Exp Ther. 2004, 309(3), 921-35; CNS Neurol Disord Drug Targets 2006, 5(1), 25-43).

Des trans-3,4,4a,5,6,10b-hexahydro-2H-napht[1,2-b]-1,4-oxazines disubstituées ont été décrites comme ligands dopaminergiques dans le brevet EP 0 899 267.

Les composés de la présente invention se comportent comme des ligands préférentiels des récepteurs D₃ avec une moindre affinité pour le récepteur D₂.

Cette caractéristique confère un intérêt tout particulier aux composés de la présente invention dû à leur faible niveau d'expression d'effets secondaires.

Plusieurs tests confirment leur mécanisme d'action et l'intérêt de leur utilisation dans le traitement de nombreuses maladies du système nerveux central.

En particulier, les composés de l'invention démontrent leur activité dans le test d'activation des autorécepteurs présynaptiques dopaminergiques, dans le test de la nage forcée, dans le test de vocalisations ultrasoniques et dans le test de rotation chez les rats lésés au 6-OH-DA.

Ces résultats permettent de proposer les produits de l'invention dans la neuroprotection et le traitement des maladies du système nerveux central dans lesquelles le système dopaminergique est impliqué, telles que la maladie de Parkinson *(*Pharmacol Ther. 2001, 90(2-3), 231-59; J Pharmacol Exp Ther. 2004, 309(3), 936-50; CNS Neurol Disord Drug Targets 2006, 5(1), 25-43), l'hyperprolactinémie *(*Pharmacol Ther. 2001, 90(2-3), 231-59; Curr Opin Obstet Gynecol. 1993, 5(3), 360-7), la dysfonction sexuelle *(*Physiol Behav. 2004, Vol 83, 291-307; J Neurosci. 1999, Vol 19, 456-463), la dépression (Pharmacol Ther. 2006, 110(2), 135-370; J Pharmacol Exp Ther. 2004, 309(3), 936-50), l'anxiété (Prog Neurobiol. 2003, 70(2), 83-244; J Pharmacol Exp Ther. 2004, 309(3), 936-50), la maladie d'Alzheimer et d'autres maladies neurodégénératives comme les attaques cérébrales (Eur J Neurosci. 2005, 22(10), 2422-30; Glia. 2005, 52(4), 336-43; J Neurosci. 2006, 26(27), 7272-80; Brain 1999, 122(Pt8), 1449-68); J Neurosci Res. 2002, 67(4), 494-500).

La présente invention a également pour objet le procédé de préparation des composés de formule I, à partir d'un composé de formule II, de configuration relative trans : 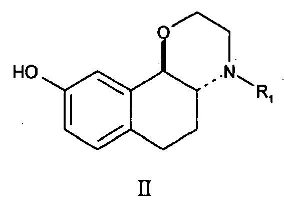 dans laquelle R₁ est tel que défini dans la formule I,
qui est mis en réaction avec de l'anhydride triflique en présence de pyridine pour conduire à un composé de formule III : 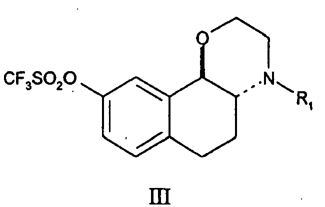 dans laquelle R₁ est tel que défini précédemment, qui est mis en réaction avec du cyanure de zinc et du tétrakis (triphénylphosphine)palladium (o) dans le diméthylformamide à chaud, pour conduire au composé de formule IV : 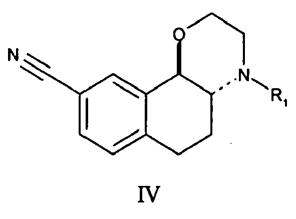 dans laquelle R₁ est tel que défini précédemment,
qui est traité par un mélange d'acide chlorhydrique et d'acide acétique au reflux, pour conduire à un composé de formule V: 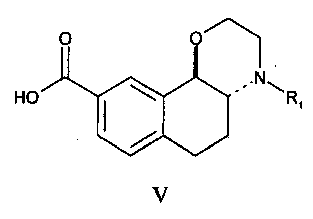 dans laquelle R₁ est tel que défini précédemment,
qui est ensuite transformé en composé de formule I, par des réactions classiques de la chimie organique.

A titre d'exemple, les composés de formule I pour lesquels X représente NH et Y représente CH₂ peuvent être obtenus par réaction d'un composé de formule V avec de la diéthylamine, dans des conditions classiques d'amidification,
pour conduire à un composé de formule VI : 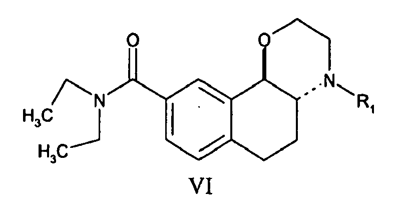 dans laquelle R₁ est tel que précédemment défini, que l'on met en réaction avec du phénylcyanate dans des conditions d'orthométallation, pour conduire à un composé de formule VII : 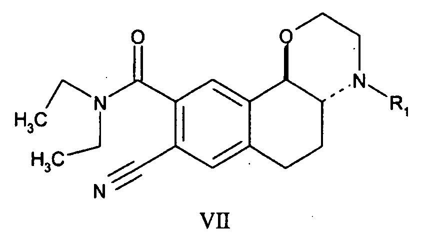 dans laquelle R₁ est tel que précédemment défini,
que l'on réduit à l'aide d'un agent réducteur classique tel que par exemple le nickel de Raney, pour conduire à un composé de formule VIII : 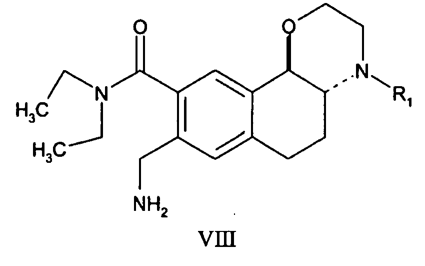 dans laquelle R₁ est tel que précédemment défini,
que l'on cyclise en présence d'un dérivé organique du lithium tel que le *tert-* butyllithium, pour conduire aux composés de formule Ia, cas particulier des composés de formule I : 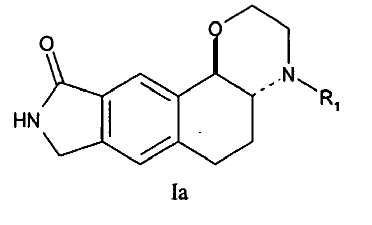 dans laquelle R₁ est tel que précédemment défini.

Les composés de formule I pour lesquels X représente O et Y représente CH₂ peuvent être obtenus par réaction d'un composé de formule VI avec du diméthylformamide, dans des conditions d'orthométallation, pour conduire à un composé de formule IX : 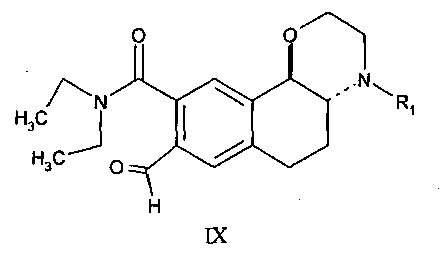 dans laquelle R₁ est tel que précédemment défini,
que l'on réduit à l'aide d'un agent réducteur sélectif tel que le borohydrure de sodium, pour conduire à un composé de formule X : 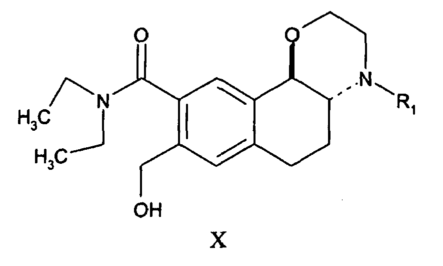 dans laquelle R₁ est tel que précédemment défini,
que l'on cyclise en présence d'un acide organique ou minéral tel que l'acide chlorhydrique, pour conduire aux composés de formule Ib, cas particulier des composés de formule I : 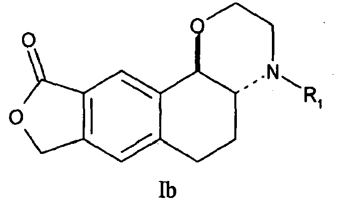 dans laquelle R₁ est tel que précédemment défini.

Les composés de formule I pour lesquels X représente un groupement NR'₂, où R'₂ représente un groupement choisi parmi alkyle C₁-C₆ linéaire ou ramifié, cycloalkyle C₃-C₈ et cycloalkylalkyle, dans lequel la partie alkyle est en C₁-C₆ et est linéaire ou ramifiée, et la partie cycloalkyle est en C₃-C₈, et Y représente un groupement CH₂, peuvent être obtenus par réaction d'un composé de formule Ib avec une amine primaire de formule NH₂R'₂, pour conduire aux composés de formule Ic, cas particulier des composés de formule I : 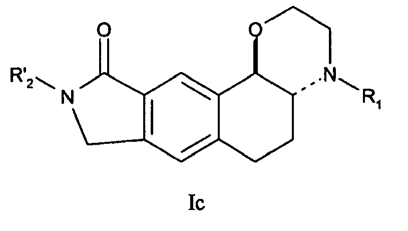 dans laquelle R₁ et R'₂ sont tels que définis précédemment.

Les composés de formule I pour lesquels X représente NH et Y représente -(CH₂)₂- peuvent être obtenus par réaction d'un composé de formule X avec un agent d'halogénation tel que le chlorure ou bromure de thionyle, ou un composé de formule CG₄ en présence de PPh₃, où G représente un atome de chlore, de brome ou d'iode,
pour conduire à un composé de formule XI : 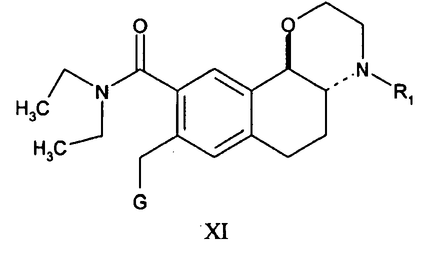 dans laquelle R₁ est tel que précédemment défini, et G représente un atome de chlore, de brome ou d'iode,
que l'on met en réaction avec un agent de cyanation tel que le cyanure de tétrabutyl ammonium ou le cyanure de sodium ou de potassium, pour conduire à un composé de formule XII : 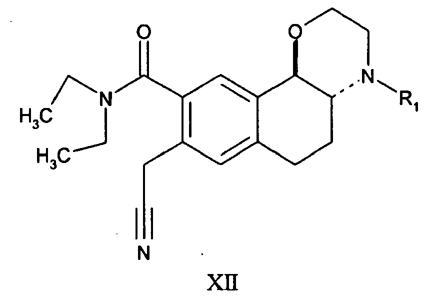 dans laquelle R₁ est tel que précédemment défini,
que l'on réduit à l'aide d'un agent réducteur classique tel que le nickel de Raney, pour conduire à un composé de formule XIII : 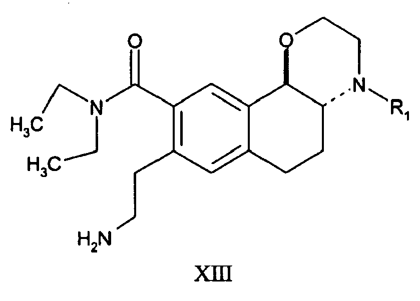 dans laquelle R₁ est tel que précédemment défini,
que l'on cyclise à l'aide d'un dérivé organique du lithium tel que le *tert*-butyllithium, pour conduire à un composé de formule Id, cas particulier des composés de formule I : 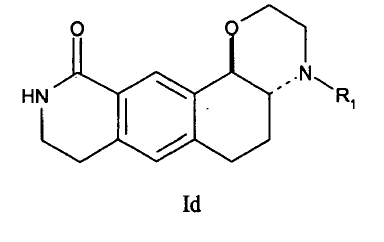 dans laquelle R₁ est tel que précédemment défini.

Les composés de formule 1 pour lesquels X représente O et Y représente -(CH₂)₂- peuvent être obtenus par réaction d'un composé de formule VI avec du bromoéthanol en présence de n-butyllithium dans des conditions d'orthométallation, pour conduire à un composé de formule XIV: 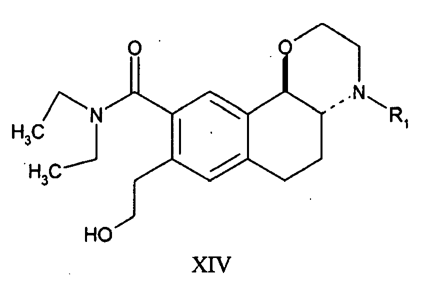 dans laquelle R₁ est tel que précédemment défini,
que l'on cyclise à l'aide d'un acide organique ou minéral tel que l'acide chlorhydrique, pour conduire aux composés de formule Ie, cas particulier des composés de formule I : 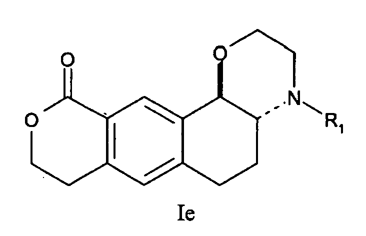 dans laquelle R₁ est tel que précédemment défini.

Les composés de formule I pour lesquels X représente un groupement NR'₂, où R'₂ représente un groupement choisi parmi alkyle C₁-C₆ linéaire ou ramifié, cycloalkyle C₃-C₈ et cycloalkylalkyle, dans lequel la partie alkyle est en C₁-C₆ et est linéaire ou ramifiée, et la partie cycloalkyle est en C₃-C₈, et Y représente un groupement -(CH₂)₂-, peuvent être obtenus par réaction d'un composé de formule Ie avec une amine primaire de formule NH₂R'₂, pour conduire aux composés de formule If, cas particulier des composés de formule I : 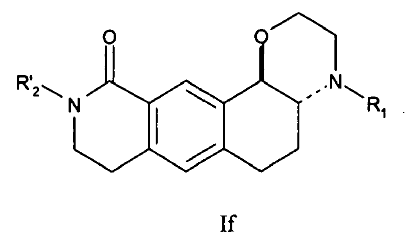 dans laquelle R₁ et R'₂ sont tels que définis précédemment.

Les composés de formule 1 pour lesquels X représente NH et Y représente CH=CH peuvent être obtenus par réaction d'un composé de formule V avec un agent chlorant tel que le chlorure de thionyle, pour conduire à un composé de formule XV : 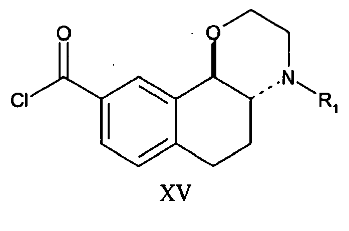 dans laquelle R₁ est tel que précédemment défini,
que l'on met en réaction avec du chlorhydrate de méthoxylamine en présence d'une base telle que du carbonate de potassium ou de sodium, pour conduire à un composé de formule XVI : 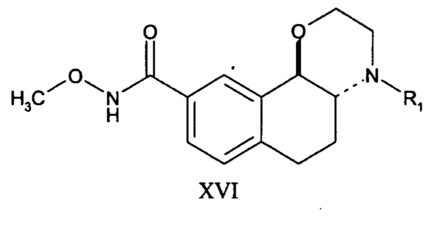 dans laquelle R₁ est tel que précédemment défini,
que l'on met en réaction avec de l'iodure de méthyle dans des conditions d'orthométallation, pour conduire à un composé de formule XVII : 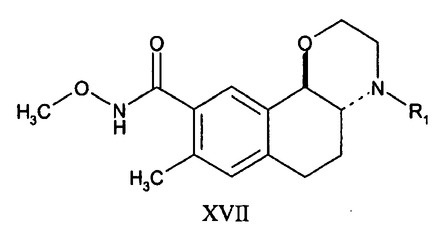 dans laquelle R₁ est tel que précédemment défini,
que l'on met en réaction avec du diméthylformamide en présence d'un dérivé organique du lithium tel que le *sec*-butyllithium, pour conduire à un composé de formule XVIII : 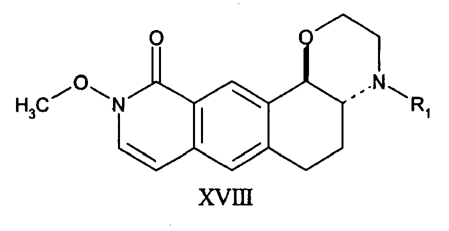 dans laquelle R₁ est tel que précédemment défini,
que l'on met en réaction avec du chlorure de titane (III), pour conduire aux composés de formule Ig, cas particulier des composés de formule I : 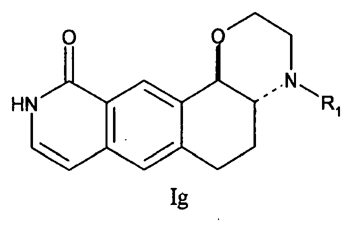 dans laquelle R₁ est tel que précédemment défini.

Les matières premières de formule II sont préparées selon des procédés décrits dans la littérature, à partir de substances connues.

Par isomère (4a*RS*, 11b*RS*) ou (4a*RS*, 10b*RS*), on entend mélange racémique des énantiomères de configurations absolues (4a*R*,11b*R*) et (4a*S*,11b*S*), ou (4a*R*,10b*R*) et (4a*S*,10b*S*), respectivement.

Les formes optiquement actives des composés de formule I sont obtenues, soit à partir de formes optiquement actives des matières premières de formule II, soit par dédoublement des formes racémiques des composés de formule I, selon des méthodes connues de la littérature.

Les composés de la présente invention sont des ligands dopaminergiques. Ils sont utiles, en tant que médicament, pour le traitement des maladies du système nerveux central dans lesquelles le système dopaminergique est impliqué, telles que la maladie de Parkinson, l'hyperprolactinémie, la dysfonction sexuelle, la dépression, l'anxiété, la maladie d'Alzheimer et d'autres maladies neurodégénératives comme les attaques cérébrales.

La présente invention a également pour objet les compositions pharmaceutiques contenant comme principe actif un composé de formule I, ou son sel d'addition à un acide pharmaceutiquement acceptable, en combinaison avec un ou plusieurs excipients ou véhicules inertes, non toxiques, pharmaceutiquement acceptables.

Parmi les compositions pharmaceutiques selon l'invention, il sera cité plus particulièrement celles qui conviennent pour l'administration orale, parentérale (intraveineuse, intramusculaire ou sous-cutanée), per ou transcutanée, nasale, rectale, perlinguale, oculaire ou respiratoire, et notamment les comprimés simples ou dragéifiés, les comprimés sublinguaux, les gélules, les capsules, les suppositoires, les crèmes, les pommades, les gels dermiques, les préparations injectables ou buvables, les aérosols, les gouttes oculaires ou nasales.

La posologie utile varie selon l'âge et le poids du patient, la voie d'administration, la nature et la sévérité de l'affection, et la prise de traitements éventuels associés et s'échelonne de 0,5 mg à 500 mg en une ou plusieurs prises par jour.

Les exemples suivants illustrent la présente invention. Les structures des composés décrits dans les exemples ont été déterminées selon les techniques spectrophotométriques usuelles (infrarouge, résonance magnétique nucléaire, spectrométrie de masse).

### EXEMPLE 1 : (4aRS,11b RS) 4-Propyl 3,4,4a,5,6,8,9,11b-octahydroisoindolo[5,6-h][1,4]benzoxazin-10(2H)-one et son chlorhydrate

### Stade A: (4aRS, 10bRS) N,N-Diéthyl 4-propyl 3,4,4a,5,6,10b-hexahydro-2H-naphto[1,2-b][1,4]oxazine 9-carboxamide

A 51 g (163 mmol) du chlorhydrate de l'acide trans (4a*RS*,10b*RS*) 4-propyl-3,4,4a,5,6,10b-hexahydro-2*H*-naphto[*1,2-b*][1,4]oxazine 9-carboxylique (préparé selon le mode opératoire décrit dans le brevet EP 0 899 267), en suspension dans du chlorure de méthylène (815 ml), sont ajoutés successivement la diéthylamine (18,3 ml, 177 mmol, 1,09 éq), le O-benzotriazol-1-yl-N, N, N', N'-tétraméthyluronium tétrafluoroborate (TBTU) (57 g, 177 mmol, 1,07 éq.) puis la triéthylamine (56 ml, 402 mmol, 2,4 éq). La solution résultante est agitée à température ambiante pendant 20 heures puis le milieu réactionnel est traité par une solution de soude 1N (425 ml). La phase organique est décantée, lavée par une solution de NaCl saturée, séchée sur sulfate de magnésium puis concentrée sous vide. Le résidu obtenu est purifié par chromatographie éclair sur gel de silice en utilisant un mélange éluant chlorure de méthylène/éthanol (90/5). Le produit attendu est récupéré sous la forme d'une huile.
*IR* : 1629 cm⁻¹
*R.M.N. ¹H 300MHz (CDCl₃)* :7.60 ; 7.25 ; 7.10 ; 4.30 ; 4.10 ; 3.95 ; 3.7-3.15 ; 3.00-2.75 ; 2.50 ; 2.4-2.2 ; 1.7-1.4 ; 1.35-1.00 ; 0.95.

### Stade B: (4aRS ,10bRS) N,N-Diéthyl 8-cyano 4-propyl 3,4,4a,5,6,10b-hexahydro-2H-naphto[1,2-b][1,4]oxazine 9-carboxamide

L'amide obtenu au stade précédent (12 g, 36 mmol) en solution dans du tétrahydrofurane (220 ml) est ajouté sur une solution de s-BuLi 1,3 M dans l'hexane (40 ml) et de N, N, N', N'-tétraméthyléthylènediamine (TMEDA) (8,2 ml) dans du tétrahydrofurane (240 ml) refroidie à-78°C, en maintenant la température interne inférieure à -65°C. Le milieu résultant est agité pendant 1h30 à une température de -70°C. Le phénylcyanate (PhOCN) (12 g) est ajouté en maintenant la température interne inférieure à -65°C. Le mélange est agité 5 minutes à -65°C, puis le milieu réactionnel est ramené à température ambiante en 1h30 puis agité à température ambiante pendant 1 heure. On hydrolyse par une solution tétrahydrofurane / eau 90/10, extrait à l'éther éthylique, séche et concentre. Le résidu obtenu est purifié par chromatographie éclair sur gel de silice en utilisant un mélange éluant chlorure de méthylène/éthanol (95/5). Le produit attendu est obtenu sous la forme d'une huile.
*IR :* 2228 cm⁻¹; 1632 cm⁻¹.
*R.M.N. ¹H 300MHz (CDCl₃)* : 7.65; 7.40; 4.30; 4.05; 3.90; 3.60; 3.25; 3.05-2.75; 2.50-2.15; 1.8-1.4; 1.15; 0.95.

### Stade C: (4aRS ,10bRS) 8-(Aminométhyl) N,N-diéthyl 4-propyl 3,4,4a,5,6,10b-hexahydro-2H-naphto[1,2-b][1,4]oxazine 9-carboxamide

Le nitrile obtenu au stade précédent (0,61g, 1,7 mmol) en solution dans du méthanol (60 ml) est traité par de l'hydrogène sous une pression de 4 bars en présence de Ni de Raney (1 g), à 60°C pendant 4h. Après retour à température ambiante, le catalyseur est filtré, le filtrat est alors concentré. Le résidu obtenu est purifié par chromatographie éclair sur gel de silice en utilisant un mélange éluant chlorure de méthylène/éthanol/ammoniaque (90/10/1). Le produit attendu est obtenu sous la forme d'un solide amorphe.
*IR* : 3388-3314 cm⁻¹, 1626 cm⁻¹.
*R.M.N. ¹H (CDCl₃) :* 7.35, s, 1H; 7.10, s, 1H; 4.30, d, 1H ; 4.05, dd , 1H; 3.90, dt, 1H ; 3.75, s, 2H ; 3.55, q, 2H; 3.20, q, 1H ; 2.85, m, 1H ; 2.90, m, 2H; 2.80, m , 1H; 2.30, m, 1H; 2.30, m , 1H; 1.55,m, 1H ;1.50, m, 2H ; 1.30, t , 3H ; 1.05, t, 3H ; 0.90, t, 3H.

### Stade D: (4aRS ,11b RS) 4-Propyl-3,4,4a,5,6,8,9,11b-octahydroisoindolo[5,6-h][1,4] benzoxazin-10(2H)-one et son chlorhydrate

Une solution de *tert*-butyllithium (1,5M dans le pentane) (10 ml) est ajoutée sur une solution de l'amine obtenue au stade précédent (1,8g) dans du tétrahydrofurane (200 ml). Le milieu résultant est agité 15 minutes à -75°C, 20 minutes à -40°C puis hydrolysé par une solution tétrahydrofurane / eau 90/10. Après décantation en présence de chlorure de méthylène, séchage et concentration sous pression réduite, le produit attendu est isolé sous la forme d'un solide blanc dont le chlorhydrate est cristallisé dans le méthanol.
*IR* :1691 cm⁻¹; 3183 cm⁻¹.
*R.M.N. ¹H 300 MHz (CDCl₃)* : 8.09; s; 1H; 7.16; s; 1H; 6.68; massif; 1H; 4.38^{;} m; 1H; 4.35; d; 1H; 4.09; m; 1H; 3.95; td; 1H; 3.00; m; 2H; 2.89; d; 1H; 2.82; m; 1H; 2.46; td; 1H; 2.29^{;} m; 3H; 1.64; m; 1H; 1.53; m; 2H; 0.92; t; 3H.

### EXEMPLE 2 : (4aR, 11bR) 4-Propyl-3,4,4a,5,6,8,9,11b-octahydroisoindolo[5,6-h][1,4] benzoxazin-10(2H)-one et son chlorhydrate

700 mg du produit obtenu au stade D de l'exemple 1 sont déposés sur une colonne Chiralcel^{®} OD et séparé par HPLC, avec pour phase mobile un mélange 200 pour 1 d'isopropanol et d'acide trifluoroacétique. Le produit attendu est le premier à être élué . Après traitement par de la soude, puis par une solution 2M d'éther chlorhydrique, on obtient le chlorhydrate du produit attendu.
*Point de fusion :* 287-291 °C
*Pouvoir rotatoire*:
- solvant: = méthanol
- conc.: = 1%
- temp.: = 20°C
- λ.: = 589nm
- D: = +52.4

### EXEMPLE 3 : (4aS, 11bS) 4-Propyl-3,4,4a,5,6,8,9,11b-octahydroisoindolo [5,6-h][1,4] benzoxazin-10(2H)-one et son chlorhydrate

Le deuxième produit élué à l'exemple 2 correspond au produit attendu. Après traitement par de la soude, puis par une solution 2M d'éther chlorhydrique, on obtient le chlorhydrate du produit attendu.
*Point de fusion*: 302-308°C
*Pouvoir rotatoire*:
- solvant: = méthanol
- conc.: = 1%
- temp.: = 20°C
- λ.: = 589nm
- D: = -53.9

### EXEMPLE 4 : (4aR ,11bR) 4-Propyl 2,3,4,4a,5,6,8,11b-octahydro-10H-furo[3',4':6,7] naphto[1,2-b][1,4]oxazin-10-one et son chlorhydrate

### Stade A: (4aR ,10bR) N,N-Diéthyl 4-propyl 3,4,4a,5,6,10b-hexahydro-2H-naphto[1,2-b][1,4]oxazine 9-carboxamide

Le chlorhydrate de l'acide (4a*R*, 10b*R*) N,N-diéthyl 4-propyl 3,4,4a,5,6,10b-hexahydro-2H-naphto[1,2-b][1,4]oxazine 9-carboxylique (α _{D} = + 90.6, à 20°C, à 1% dans le méthanol) est traité comme au stade A de l'exemple 1 pour conduire au produit attendu.

### Stade B: (4aR, 10bR) N,N-Diéthyl 8-formyl 4-propyl 3,4,4a,5,6,10b-hexahydro-2H-naphto[1,2-b][1,4]oxazine 9-carboxamide

L'amide obtenu au stade précédent (2 g, 6,05 mmol), en solution dans du tétrahydrofurane (10 ml) est ajouté sur une solution de s-BuLi 1,3 M (7,86 ml) et de N, N, N', N'-tétraméthyléthylènediamine (TMEDA) (1,2 ml, 7,9 mmol) dans du tétrahydrofurane (25 ml) refroidi à - 78°C, en maintenant la température toujours inférieure à -65°C. Le milieu résultant est agité pendant 1h30 puis la *N,N*-diméthylformamide (1 ml) est ajoutée en maintenant la température interne inférieure à -65°C. Le mélange est agité 5 minutes à -65°C, puis le milieu réactionnel est ramené à température ambiante en 1h30 et agité pendant 1 heure à cette température.
Apres hydrolyse par une solution d'eau à 10% dans du tétrahydrofurane, extraction à l'éther éthylique, séchage et concentration, le résidu obtenu est purifié par chromatographie éclair sur gel de silice en utilisant un mélange éluant (chlorure de méthylène/éthanol : 95/5). Le produit attendu est récupéré sous la forme d'une huile.
*IR* :2940-1696 cm⁻¹ ; 1628 cm⁻¹.
*R.M.N. ¹H 300MHz (CDCl₃)* : 9.90, s, 1H ; 7.70, s , 1H ; 6.30, s, 1H; 4.25, d, 1H; 4.00, m , 1H; 3.80 , m, 1H ; 3.45, q , 2H ; 3.00, q, 2H ; 3.00-2.70, m, 4H ; 2.35-2.05, m, 4H; 1.45, m, 3H; 1.20, t , 3H; 0.90, t, 3H ; 0.85, t, 3H .

### Stade C: (4aR ,11bR) 4-Propyl 2,3,4,4a,5,6,8,11b-octahydro-10H-furo[3',4':6,7] naphto[1,2-b][1,4]oxazin-10-one et son chlorhydrate

L'aldéhyde obtenu au stade précédent (3,5 g) est dissout dans du méthanol (35 ml). La solution refroidie à 0°C est traitée par du borohydrure de sodium (0,45 g). Le milieu réactionnel est agité 20 heures avec retour à température ambiante. Le milieu réactionnel est refroidi à 0°C, puis une solution d'acide chlorhydrique 6N (7 ml) est ajouté. Le milieu résultant est chauffé au reflux pendant 20 heures. Apres retour à température ambiante, le chlorhydrate du produit attendu est isolé et recristallisé dans le méthanol.
*Point de fusion* : 268-271°C
*IR* : 2780 cm⁻¹, 2140 cm⁻¹, 1746 cm⁻¹.
*R.M.N. ¹H 300MHz (DMSO-d6) :*
11.90, m, 1H; 7.80, s, 1H; 7.50, s, 1H; 5.40, s, 2H; 5.10, d, 1H; 4.25, m, 2H; 3.60, m, 1H; 3.45-3.15, m, 3H; 3.15-2.95, m, 3H; 2.55, m, 1H; 2.10, m, 1H;1.75 (sext), 2H; 0.95 (t), 3H.

### EXEMPLE 5 : (4aR ,12bR) 4-Propyl 3,4,4a,5,6,8,9,12b-octahydro-2H,11H-pyrano[4',3':6,7] naphto[1,2-b][1,4]oxazin-11-one et son chlorhydrate

### Stade A: (4aR , 10bR) 8-(2-Hydroxyéthyl) N,N-diéthyl 4-propyl 3,4,4a,5,6,10b-hexahydro-2H naphto[1,2-b][1,4]oxazine 9-carboxamide

L'amide obtenu au stade A de l'exemple 4 (5 g, 15 mM), en solution dans du tétrahydrofurane (60 ml) est ajouté sur une solution de s-BuLi 1,3 M dans l'hexane (14,7 ml) et de N, N, N', N'-tétraméthyléthylènediamine (TMEDA) (3 ml) dans du tétrahydrofurane (65 ml) refroidi à-78°C, en maintenant la température interne inférieure à -65°C. Le milieu résultant est agité pendant 30 minutes à une température de -70°C. Une solution de lithioéthanolbromé [préparée à partir de bromoéthanol et de n-BuLi (2,5 M dans l'hexane) dans le tétrahydrofurane] est transférée par canulation en maintenant la température interne inférieure à -65°C. le mélange est agité 5 minutes à -65°C, puis le milieu réactionnel est ramené à température ambiante en 1h30 et l'agitation est maintenue encore pendant 1 heure. On hydrolyse par une solution à 10% d'eau dans du tétrahydrofurane, extrait au chlorure de méthylène, sèche et concentre . Le résidu obtenu est purifié par chromatographie éclair sur gel de silice en utilisant un mélange éluant chlorure de méthylène/éthanol (90/10). Le produit attendu est isolé sous la forme d'une huile.
*IR* : 3600-3090 cm⁻¹; 1621 cm⁻¹.
*R.M.N. ¹H 300MHz (DMSO-d6)* : 7.10, s , 1H ; 7.00, s, 1H ; 4.60, t , 1H ; 4.15, d, 1H ; 3.95, d , 1H ; 3.75 , t , 1H ; 3.50, m , 2H ; 3.40, m, 2H ; 3.05, m, 2H ; 2.80, m, 2H ; 2.60, m , 2H ; 2.30-2.00, m, 5H ; 1.55-1.35, m, 4H ; 1.15, t , 3H ; 0.95, t, 3H ; 0.85, t, 3H .

### Stade B: (4aR , 12bR) 4-Propyl 3,4,4a,5,6,8,9,12b-octahydro-2H,11H-pyrano[4',3':6,7] naphto[1,2-b][1,4]oxazin-11-one et son chlorhydrate

Une solution d'acide chlorhydrique 6N (2,1 ml) est ajouté a température ambiante à une solution du produit obtenu au stade précédent (1,08 g, 2,88 mmol) dans le méthanol (9 ml). Le milieu résultant est chauffé au reflux pendant 20 heures. Après retour à température ambiante, la filtration du précipité formé permet d'obtenir le chlorhydrate du produit attendu.
*Point de fusion* : 275-279°C
*IR* : 2401 cm⁻¹, 1712 cm⁻¹, 1620 cm⁻¹.
*R.M.N. ¹H 300MHz (DMSO-d6) :* 11.50, m, 1H; 8.00, s, 1H; 7.21, s, 1H; 4.95, dd, 1H; 4.50, t, 2H ; 4.25, d, 2H; 3.60, m, 1H; 3.28, m, 3H; 3.05, m, 5H; 2.50, m, 1H ; 2.00, m, 1H; 1.75, m, 2H; 1.00, t, 3H.

### EXEMPLE 6 : (4aR,12bR) 4-Propyl-2,3,4,4a,5,6,8,9,10,12b-décahydro-11H isoquino [6,7-h][1,4]benzoxazin-11-one et son chlorhydrate

### Stade A: (4aR, 10bR) 8-Hydroxyméthyl N,N-diéthyl 4-propyl 3,4,4a,5,6,10b-hexahydro-2H-naphto[1,2-b][1,4]oxazine 9-carboxamide

L'aldéhyde obtenu au stade B de l'exemple 4 (0,85 g, 2,4 mmol) est dissout dans du méthanol (10 ml). La solution refroidie à 0°C est traité par du borohydrure de sodium (0,16 g, 4,23 mmol). Le milieu réactionnel est agité 20 heures avec retour à température ambiante. Le méthanol est évaporé sous vide. Le résidu est repris par de l'eau et du chlorure de méthylène. Apres décantation, séchage et concentration, le résidu obtenu est purifié par chromatographie éclair sur gel de silice en utilisant un mélange éluant chlorure de méthylène/éthanol (95/5). Le produit attendu est obtenu sous la forme d'une huile.
*IR* : 3600-3070 cm⁻¹; 1627 cm⁻¹.
*R.M.N. ¹H 300MHz (DMSO-d6)* : 7.20, s , 1H ; 7.10, s, 1H ; 5.10, t , 1H ; 4.35, d, 1H ; 4.20, d , 1H ; 4.00 , m, 1H ; 3.80, m , 1H ; 3.40, q, 2H ; 3.10, q , 2H ; 2.80, m , 4H ; 2.40-2.00, m , 4H ; 1.45, m, 3H ; 1.10, t , 3H ; 0.95, t, 3H ; 0.85, t, 3H .

### Stade B: (4aR , 10bR) 8-Chlorométhyl N,N-diéthyl 4-propyl 3,4,4a,5,6,10b-hexahydro-2H-naphto[1,2-b][1,4]oxazine 9-carboxamide

L'alcool obtenu au stade précédent, en solution dans du toluène, est traité par du chlorure de thionyle (0,4 ml). Le milieu est agité à température ambiante pendant 20 heures. Le toluène est évaporé sous vide. Le résidu est repris par de l'eau et du chlorure de méthylène. Après décantation, lavage par une solution aqueuse de bicarbonate de sodium, séchage, le composé attendu est obtenu sous la forme d'une huile.
*IR* : 1627 cm⁻¹.
*R.M.N. ¹H 300MHz (DMSO-d6) :* 7.25, s , 1H ; 7.20, s, 1H ; 4.65, d, 1H ; 4.20, d , 1H ; 4.00 , dd, 1H ; 3.80, td, 1H ; 3.45, q, 2H ; 3.10, q , 2H ; 2.90-2.70, m, 4H ; 2.40-2.05, m , 4H ; 1.6-1.35, m, 3H ; 1.25, t , 3H ; 1.00, t, 3H ; 0.90, t, 3H .

### Stade C: (4aR , 10bR) 8-Cyanométhyl N,N-diéthyl 4-propyl 3,4,4a,5,6,10b-hexahydro-2H-naphto[1,2-b][1,4]oxazine 9-carboxamide

Le composé obtenu au stade précédent (0,64 g, 1,68 mmol) en solution dans du tétrahydrofurane (15 ml) est traité par du cyanure de tétra-butylammonium (0,8 g, 2,98 mmol) pendant 20 heures. Le milieu est concentré sous vide. Le résidu est repris par de l'eau et du chlorure de méthylène. Après décantation, séchage puis concentration, le nitrile attendu est obtenu sous la forme d'une huile.
*IR* :1628 cm⁻¹.
*R.M.N. ¹H 300MHz (DMSO-d6)* : 7.20, 2s , 2H ; 4.20, d, 1H ; 4.00, dd , 1H ; 3.80, m+s , 3H ; 3.45, q, 2H ; 3.10, q, 2H ; 2.90-2.70, m, 4H ; 2.35-2.05, m, 4H ; 1.55-1.35, m, 3H ; 1.15, t , 3H; 1.00,t,3H;0.85,t,3H.

### Stade D: (4aR, 10bR) 8-(2-Aminoéthyl) N,N-diéthyl 4-propyl-3,4,4a,5,6,10b-hexahydro-2H-naphto[1,2-b][1,4]oxazine 9-carboxamide

Le composé obtenu au stade précédent (2,7 g, 7,3 mmol), en solution dans du méthanol (250 ml), est traité par de l'hydrogène sous une pression de 4 bars en présence de Ni de Raney (1 g), à 60°C, pendant 4h. Après retour à température ambiante, le catalyseur est filtré, le filtrat est alors concentré. Le résidu obtenu est purifié par chromatographie éclair sur gel de silice en utilisant un mélange éluant chlorure de méthylène/éthanol/ammoniaque (90/10/1). Le produit attendu est isolé sous la forme d'un solide amorphe.
*IR* : 3360-3310 cm⁻¹, 1626 cm⁻¹.
*R.M.N. ¹H 300MHz (DMSO-d6)* : 7.10, s, 1H; 7.00, s, 1H; 4.20, d, 1H ; 4.00, m, 1H ; 3.80, m, 1H ; 3.45, q, 2H; 3.10, q, 2H; 2.9-2.7, m, 4H; 2.50, m, 2H; 2.4-2.05, m, 4H;1.6-1.3, m, 3H; 1.20, t, 3H;1.00, t, 3H ;0.90, t, 3H .

### Stade E: (4aR, 12bR) 4-Propyl-2,3,4,4a,5,6,8,9,10,12b-décahydro-11H isoquino[6,7-h][1,4]benzoxazin-11-one et son chlorhydrate

Une solution de *tert*-butyllithium 1,5 M dans le pentane (1,9 ml, 3,21 mmol) est ajoutée sur une solution de l'amine obtenue au stade précédent (0,40 g) dans du tétrahydrofurane (45 ml). Le milieu résultant est agité 10minutes à -78°C, puis 20minutes à -40°C. Le milieu est hydrolysé par une solution à 10% d'eau dans du tétrahydrofurane. Après décantation en présence de chlorure de méthylène, séchage et concentration sous pression réduite, le résidu obtenu est purifié par chromatographie éclair sur gel de silice en utilisant un mélange éluant chlorure de méthylène/éthanol (90/10). Le produit attendu est isolé sous la forme d'un solide amorphe dont le chlorhydrate est cristallisé de l'acétate d'éthyle.
*Point de fusion :* 263-265°C
*IR* : 1666 cm⁻¹.
*R.M.N. ¹H (DMSO-d6)* : 7.90, s, 1H; 7.10, s, 1H ; 5.00, d, 1H ; 4.25, m, 2H ; 3.60, m, 1H; 3.4-3.15, m, 5H; 3.00, m, 3H; 2.85, m, 2H; 2.50, m, 2H ; 2.00, m, 1H; 1.75, m, 2H; 1.00, t, 3H.

### EXEMPLE 7 : (4aR ,11bR) 9-Méthyl 4-propyl 3,4,4a,5,6,8,9,11b-octahydroisoindolo[5,6-h][1,4]benzoxazin-10(2H)-one et son chlorhydrate

Le produit de l'exemple 4 (1g, 3,08 mmol) en solution dans une solution aqueuse de méthylamine à 40% (10 ml) est chauffée pendant 16 heures en autoclave à 120°C. Après retour à température ambiante, le milieu est extrait au chlorure de méthylène, la phase organique est séchée sur MgSO₄. La concentration conduit à un résidu qui est purifié par chromatographie éclair sur gel de silice en utilisant un mélange éluant chlorure de méthylène/éthanol (95/5). Le produit attendu est obtenu sous la forme d'un solide blanc dont le chlorhydrate est cristallisé de l'acétonitrile.
*Point de fusion* : 240-245°C
*IR* : 1692 cm⁻¹.
*R.M.N. ¹H 300MHz (DMSO-d6)* : 8.00, s, 1H ; 7.10, s, 1H ; 4.35, d, 1H ; 4,30, s ; 2H ; 4.10, m,1H ; 3.95, m, 1H ; 3.20, s, 3H ; 3.00, m, 2H ; 2.90, m, 1H ; 2.85, m, 1H ; 2.50, m,1H ; 2,30, m, 3H; 1.7-1.4 ; m, 3H ; 0.90, t, 3H.

### EXEMPLE 8 : (4aR, 12bR) 4-Propyl-2,3,4,4a,5,6,10,12b-octahydro-11H-isoquino [6,7-h][1,4]benzoxazin-11-one et son chlorhydrate

### Stade A: Chlorure de l'acide (4aR,10bR) 9-(4-propyl 3,4,4a,5,6,10b-hexahydro-2H-naphto[1,2-b][1,4]oxazine) carboxylique

3,6 ml (41,7 mmol) de chlorure de thionyle est ajouté goutte à goutte sur l'acide (4a*R*, 10b*R*) 4-propyl-3,4,4a,5,6,10b-hexahydro-2H-naphto[1,2-b][1,4]oxazine 9-carboxylique (10 g, 32 mmol) en suspension dans du toluène anhydre (100 ml) et de la diméthylformamide (0,15 ml). Le mélange résultant est chauffé à reflux pendant 1 heure. Après retour à température ambiante, le milieu est filtré, le résidu solide est lavé par du toluène. Le solide est séché à l'étuve sous vide en présence de P₂O₅ jusqu'à poids constant, pour conduire au produit attendu.
*IR*: 2457 cm⁻¹; 1753 cm⁻¹ ; 814-775 cm⁻¹.
*R.M.N. ¹H 300MHz (DMSO-d6)* : 8.05 (s) 1H, 7.80 (dd) 1H, 7.30 (d) 1H, 5.05 (d) 1H, 4.30 (m) 2H, 3.60 (d) 1H, 3.50 (NH) 1H, 3.30 (m) 3H, 3.00 (m) 3H, 2.50 (m) 1H, 2.10 (m) 1H, 1.75 (m) 2H, 0.95 (t) 3H.

### Stade B: Méthoxy méthyl amide de l'acide (4aR ,10bR) 9-(4-propyl 3,4,4a,5,6,10b-hexahydro-2H-naphto[1,2-b][1,4]oxazine) carboxylique

2,62g (31,3 mmol) de chlorhydrate de méthoxylamine est ajouté à un mélange de carbonate de potassium (13 g, 94 mmol) dans de l'eau (31 ml) et de l'acétate d'éthyle (62 ml). Au mélange refroidi à 0°C est ajouté par portion le chlorure d'acide du stade A (10,35 g, 31,3 mmol) en maintenant la température inférieure à 5°C. Le milieu est agité 2 heures à 0°C. Après ajout d'acétate d'éthyle et d'eau et retour à température ambiante, le milieu est décanté, la phase organique est lavée par de l'eau, séchée sur sulfate de magnésium, puis concentrée sous vide, pour conduire au produit attendu sous la forme d'un solide.
*Point de fusion* : 147-152°C
*IR:* 3194 cm⁻¹,2870 cm⁻¹, 2803-2767 cm⁻¹,1650 cm⁻¹,1272-1126 cm⁻¹, 834-760 cm⁻¹..
*R.M.N. ¹H 300MHz (DMSO-d6)* :11.60 (s) 1H, 7.82 (s) 1H, 7.55 (dd) 1H, 7.18 (d) 1H, 4.20 (d) 1H, 4.00 (dd) 1H, 3.80 (td) 1H, 2.7 à 3.00 (m) 3H, 2.30 (m) 2H, 2.05 à 2.2 (m) 2H, 1.45 (m) 3H, 0.88 (t) 3H.

### Stade C: (4aR, 12bR) 10-Méthoxy 4-propyl 2,3,4,4a,5,6,10,12b-octaahydro-11H- isoquino[6,7-h][1,4]benzoxazin-11-one

Une solution de l'amide obtenu au stade B (4 g, 13,14 mmol) dans le tétrahydrofurane (40ml) est ajouté à une solution de s-BuLi (24ml, 31,53mM) et de N, N, N', N'-tétraméthyléthylènediamine (TMEDA) (4,8ml, 31,53 mmol) dans le tétrahydrofurane (90 ml) refroidie à -78°C, en maintenant une température inférieure à -70°C. Le milieu réactionnel est ramené à une température de -20°C, puis agité pendant 45 minutes à une température moyenne de -10°C. Le milieu est refroidi à nouveau à -78°C et de l'iodure de méthyle (0,9 ml, 14,45 ml) est ajouté. La température est ramenée à 0°C puis à température ambiante. L'hydrolyse est réalisée par une solution saturée de chlorure d'ammonium. Apres ajout d'éther éthylique, le milieu est décanté, la phase organique est lavée par de l'eau, séchée sur sulfate de magnésium, puis concentrée sous vide. Une solution de s-BuLi (21,4 ml, 27,8 mmol) est ajoutée à une solution du résidu obtenu (4,04 g) dans le tétrahydrofurane (83 ml). On refroidit à -78°C et agite pendant 2 heures à cette température . De la diméthylformamide (1,13 ml, 14,6 mmol) est ajoutée au milieu, en maintenant une température inférieure à -70°C, qui est agité pendant 10 minutes à cette température avant d'être ramené à température ambiante. L'hydrolyse est réalisée par une solution saturée de chlorure d'ammonium. Après ajout d'éther éthylique, le milieu est décanté, la phase organique est lavée par de l'eau, séchée sur sulfate de magnésium, puis concentrée sous vide. Le résidu est repris par du tétrahydrofurane (330 ml), de l'acide chlorhydrique concentré est ajouté (13,5 ml) et le mélange est agité pendant 1 heure à température ambiante. Après traitement par une solution de soude concentrée, extraction par de l'acétate d'éthyle, lavages par de l'eau et par une solution de chlorure de sodium, la phase organique est séchée sur sulfate de magnésium, puis concentrée sous vide. Le résidu obtenu est purifié par chromatographie éclair sur gel de silice en utilisant un mélange éluant chlorure de méthylène/éthanol (95/5). Le produit attendu est isolé sous la forme d'un solide beige.
*Point de fusion* : 142-145°C
*R.M.N. ¹H 400MHz (CDCl₃)* : 8.65, 7.25, 7.22, 6.35, 4.47, 4.12-4.03, 4.05, 3.00 ppm

### Stade D: (4aR, 12bR) 4-Propyl 2,3,4,4a,5,6,10,12b-octahydro-11H- isoquino [6,7-h][1,4]benzoxazin-11-one et son chlorhydrate

Une solution de TiCl₃ à 15% dans l'eau (7,6 ml) est ajouté a une solution du composé du stade C (1,1 g, 3,35 mmol) dans de l'éthanol (3,3 ml). Le milieu réactionnel est chauffé pendant 24 heures à 45°C. Le chauffage est poursuivi pendant 6 jours avec ajout d'une une solution de TiCl₃ à 15% (3,5 ml) quotidiennement. Après retour à température ambiante, le milieu est traité par de l'eau (30 ml) et de la glace (30 g), puis alcalinisé à pH 13-14 par de la soude à 35%. La suspension noire est traitée par un courant d'air comprimé jusqu'à décoloration totale. Après extraction au chlorure de méthylène, séchage et concentration, le résidu obtenu est purifié par chromatographie éclair sur gel de silice en utilisant un mélange éluant chlorure de méthylène/éthanol (95/5). Le produit attendu est isolé sous la forme solide blanc dont le chlorhydrate est cristallisé dans l'acétonitrile.
*Point de fusion* : 200-203°C
*IR:* 3457 cm⁻¹,3162 cm⁻¹,1632 cm⁻¹.
*R.M.N. ¹H 400MHz (DMSO-d6) :*11.10 (m) 2H, 8.25 (s) 1H, 7.40 (s) 1H, 7.10 (t) 1H, 6.40 (d) 1H, 4.95 (d) 1H, 4.25 (m) 2H, 3.60 (d) 1H, 3.3-3.0 (2m) 2H, 3.05-2.00 (2m) 4H, 1.70 (m) 2H, 0.95 (t) 3H.

### ETUDE PHARMACOLOGIQUE

### EXEMPLE 9 : Etude de la liaison aux récepteurs humains D₂ et D₃

### ◆ Culture cellulaire

Les cellules CHO (Chinese Hamster Ovary) ont été transfectées de façon stable par le gène codant pour le récepteur humain de la dopamine D₂ ou D₃ selon les méthodes connues de la littérature. Les cellules natives sont déficientes en enzyme DHFR (DiHydroFolate Reductase). Ces cellules sont cultivées dans une étuve à 37° C en atmosphère humide 5 % CO₂, 95 % air. Les transfections ont été réalisées en utilisant la Lipofectine (Gibco). Les cellules CHO cotransfectées avec le récepteur D₂ humain et le gène de résistance à la phléomycine ont été sélectionnées pour leur résistance à la présence de cet antibiotique dans le milieu de culture. Les cellules transfectées avec le récepteur D₃ humain ont été sélectionnées dans un milieu dépourvu d'hypoxanthine/thymidine, en présence de méthotréxate. La composition des milieux de culture utilisés sont pour les CHO-D₂ : DMEM (Dulbecco's Modified Eagle Medium) supplémentés à 10 % en sérum de veau foetal et en hypoxanthine/thimidine et pour les CHO-D₃ : DMEM supplémentés à 10 % en sérum de veau foetal dialysé. Les cellules sont récoltées à confluence et les membranes sont alors préparées.

### ◆ Préparation membranaire :

Après quelques minutes en présence de trypsine 0,2 %, les cellules sont récupérées et centrifugées à 2 000 g pendant 5 minutes. Le culot cellulaire, resuspendu dans du tampon Tris-HCl 10 mM, pH 7,5 contenant 5mM de MgSO₄, est alors passé au Polytron^{®}. L'homogénat est ensuite centrifugé à 50 000g pendant 15 minutes, et le culot est resuspendu par sonication douce dans le tampon d'incubation de composition : 50 mM de tris-HCl, pH 7,5 contenant 120 mM de NaCl, 5 mM de KCl, 2 mM de CaCl₂, 5 mM de MgCl₂. Les membranes sont ensuite aliquotées et conservées à -80° C jusqu'au jour de l'expérience.

### ◆ expériences de liaison

Les incubations sont effectuées dans des tubes en polypropylène pour un volume final de 400 µl contenant :
100 µl de [¹²⁵I]-iodosulpride (Amersham)
à 0,1 et 0,2 nM pour les récepteurs D₂ et D₃ respectivement.
100 µl de tampon (tubes totaux)
ou 100 µl de raclopride 10 µM (liaison non spécifique)
ou 100 µl de composé
200 µl de préparation membranaire contenant les récepteurs D₂ ou D₃, dans du tampon additionné de 0,2 % de BSA (Bovine Serum Albumine).

Les gammes de concentration de chaque composé comportent au moins sept points déterminés en triple. Chaque expérience est répétée au moins deux fois.
L'incubation qui dure trente minutes à 30° C est stoppée par une filtration rapide sur appareil Brandle, suivie de trois rinçages consécutifs avec du tampon Tris-HCl pH 7,4 contenant 120 mM de NaCl. Les filtres récupérés sont ensuite comptés au compteur gamma.

### ◆ Analyse des résultats

L'IC₅₀ représentant la concentration donnant 50 % d'inhibition de la liaison du radioligand est calculée par régression non linéaire (méthode Prism Graph).
La valeur de Kᵢ est dérivée de la formule Kᵢ = IC₅₀/(1+L/Kd) où L est la concentration de [¹²⁵I]-iodosulpride utilisée dans l'expérience et Kd sa constante de dissociation. Les résultats sont exprimés sous la forme de pKᵢ (pKᵢ = -logKᵢ).

Pour les récepteurs humains D₂ et D₃, les Kd sont respectivement égaux à 0,5 et 1,31 nM.

### ◆ Résultats

| **Composé** | **pKᵢ** | |
|---|---|---|
| | **hD₃** | **hD₂** |
| Exemple 1 | 8,1 | 5,9 |
| Exemple 2 | 8,4 | 6,1 |
| Exemple 4 | 8,1 | 5,7 |
| Exemple 5 | 6,9 | 5,8 |
| Exemple 6 | 7,4 | 6,1 |
| Exemple 8 | 7,7 | 5,9 |

### EXEMPLE 10 : Activation des autorécepteurs présynaptiques dopaminergiques. Test d'enregistrement de l'activité électrique unitaire, extracellulaire, dans l'aire tegmentale ventrale du rat.

### ◆ Principe

L'administration d'un agoniste dopaminergique diminue d'une manière dose-dépendante la fréquence de décharge des neurones. Cet effet est réversé par l'halopéridol, antagoniste dopaminergique.

### ◆ Méthode

Les rats sont anesthésiés à l'hydrate de chloral (400 mg/kg, i.p.) et placés dans un appareil de stéréotaxie (Unimécanique, France) après cathétérisation de la veine fémorale. Le niveau d'anesthésie est maintenu par une administration i.p. de l'hydrate de chloral toutes les heures ; la température rectale est conservée à 37±1° C par une couverture chauffante thermostatée. Une microélectrode en tungstène (10 MΩ, 1µm) est descendue à l'aide d'un microdescendeur électronique (Unimécanique, France) au niveau de l'aire tegmentale ventrale (AP : -5.5 / bregma ; L : 0.7 ; H : 7.0-8.5 / dura ; atlas de Paxinos et Watson, 1986). Les potentiels de cellules dopaminergiques sont reconnus par leur morphologie (potentiels triphasiques +/-/+, d'une durée supérieure à 3 msec), leur rythme de décharge, soit régulier, soit en bouffées d'amplitude décroissante, et leur fréquence de décharge comprise entre 2 et 8 Hz. On enregistre une seule cellule par animal.

Après une période ≥ 5 min. (activité basale) et une première injection de véhicule (eau distillée additionnée de quelques gouttes d'acide lactique dilué ; pH ramené à 5 par NaOH 1N), les produits de l'invention sont administrés par voie intraveineuse en doses cumulées croissantes avec un intervalle de 2-3 min.

### ◆ Analyse des résultats

L'acquisition des données est faite par le logiciel Spike2 (Cambridge Electronic Design, England). La fréquence de décharge est mesurée sur une minute au maximum de variation entre chaque injection et exprimée en pourcentage de variation par rapport à l'activité basale (moyenne des 5 minutes précédant le premier traitement) définie comme 100 %. L'effet des produits est évalué statistiquement par une analyse de variance sur mesures répétées suivie d'un test de Dunnett pour comparaison des effets des différentes doses à l'effet du véhicule (eau distillée).

### ◆ Résultats

A titre d'exemple, le tableau suivant montre les effets du produit de l'exemple 2.

| **Exemple 2 - dose** µg/kg i.v. | **Fréquence de décharge des neurones** |
|---|---|
| véhicule (0) | 102,6 ± 0,9 |
| 0,125 | 91,8 ± 5,2 |
| 0,25 | 79,9 ±5,9* |
| 0,5 | 66,3 ± 5,9* |
| 1,0 | 42,0 ± 7,3* |
| 2,0 | 10,8 ± 8,7* |
| 4,0 | 2,3 ± 2,3* |
| 8,0 | 0,0 ± 0,0* |

| | |
|---|---|
| *Valeurs individuelles (n = 5) = Moyenne. ± erreur standard à la moyenne*. * *= p < 0,05 versus véhicule* | |

### EXEMPLE 11 : Propriétés antidépressives : test de la nage forcée chez le rat

### ◆ Principe

Le test de la nage forcée (Porsolt R. et al, Eur. J. Pharmacol., 1978, 47, 379-91) est un test comportemental qui consiste à induire chez le rat un état de "désespoir", en plaçant l'animal naïf pendant quinze minutes dans une enceinte remplie d'eau, d'où il ne peut s'échapper. Pendant les cinq à dix premières minutes, le rat se débat vigoureusement pour finalement adopter une posture immobile pendant la fin de l'essai. Placé le jour suivant dans la même enceinte, l'animal reste immobile pendant la majeure partie du test (durée 5 min). Les antidépresseurs réduisent la durée d'immobilité du rat, lors du test.

### ◆ Expérimentation

L'expérience s'effectue en deux jours, à 24 heures d'intervalle, sur des rats d'un poids moyen de 170 g, stabulés la veille en cages individuelles, avec libre accès à la boisson et à la nourriture.

Le premier jour, chaque rat est placé pendant quinze minutes, dans un cylindre en verre (20 cm de diamètre x 40 cm de haut) rempli d'eau maintenue à 25° C, sur une hauteur de 15 cm. Le deuxième jour, l'animal est placé à nouveau dans un cylindre pendant cinq minutes ; le temps total (en sec) d'immobilité du rat est mesuré. Le produit ou le solvant est administré à l'animal trente minutes avant le début du test. L'effet des produits est évalué statistiquement par une analyse de variance sur mesures répétées suivie d'un test de Dunnett pour comparaison des effets des différentes doses à l'effet du véhicule (eau distillée).

### ◆ Résultats

A titre d'exemple et pour illustrer l'activité des produits de l'invention, les effets du produit de l'exemple 2 sont rapportés dans le tableau suivant :

| **Produit** | **Dose** mg/kg s.c. | **Immobilité (sec)** moyenne ± e.s.m. |
|---|---|---|
| Véhicule (eau distillée) | 0 | 174,3 ± 9,1 |
| Exemple 2 | 0,02 | 159,4 ± 7,9 |
| | 0,04 | 122,7 ± 21,2* |
| | 0,08 | 22,03 ± 5,8* |

| | | |
|---|---|---|
| ** p<0,05 versus véhicule - e.s.m. = erreur standard par rapport à la moyenne* | | |

Le produit de l'exemple 2 réduit de façon dose-dépendante la durée d'immobilité de l'animal et présente donc un excellent effet antidépresseur.

### EXEMPLE 12 : Rotations induites par les agonistes dopaminergiques chez des rats avec lésion unilatérale de la Substantia nigra

### ◆ Principe

L'injection unilatérale de la neurotoxine 6-hydroxy-dopamine (6-OH-DA) dans la *Substantia nigra* produit une dégénérescence des voies ascendantes nigrostriées, avec hypersensibilité des récepteurs dopaminergiques post-synaptiques du côté lésé. Chez un rat ayant subi une telle lésion, l'administration systémique de produits agonistes directs (apomorphine) induit des rotations contralatérales (du côté opposé à la lésion). Ce test permet de mettre en évidence les propriétés dopaminergiques agonistes de produits à visée thérapeutique dans la maladie de Parkinson.

### ◆ Méthodes

**Lésion :** la lésion est effectuée sur des rats Wistar mâles de 280 à 330 g anesthésiés au pentobarbital (40-50 mg/kg i.p.) et ayant reçu une dose de 25 mg/kg i.p. de désipramine. L'animal est placé dans un appareil stéréotaxique KOPF, le crâne orienté selon l'Atlas de Pellegrino et Cushman (1979). Un volume de 4 µl d'une solution de 6-OH-DA (2 µg/µl) est injecté lentement au moyen d'un microperfuseur, au niveau de la *Substantia nigra* gauche, (A = 2,4 mm ; L = 2.0 mm ; V = 3.1 mm, par rapport au zéro interaural) (U. Ungerstedt, Acta Physiol. Scandi. Suppl., 1971, 367, 69-93)

**Matériel :** l'enregistrement du nombre et du sens des rotations se fait automatiquement sur ordinateur, grâce au système ROTACOUNT (Columbus Co, USA). L'animal est placé dans un cylindre à fond plat, de 30 cm de diamètre et 50 cm de hauteur. Un câble fin, semi-rigide, ceinture l'animal sous les pattes avant et se connecte à la cellule de comptage optique située au dessus du cylindre et reliée à l'ordinateur.

**Sélection des animaux lésés :** un mois après l'induction de la lésion par la 6-OH-DA, les animaux correctement lésés sont sélectionnés selon un critère d'au moins 150 rotations contralatérales effectuées en 1 heure après administration de l'agoniste dopaminergique, apomorphine (0,04 mg/kg, s.c.).

**Expérimentation :** les animaux sont testés une fois par semaine, avec administration des produits de l'invention en alternance avec celle de l'agoniste dopaminergique. L'enregistrement des rotations contralatérales débute dès l'injection de l'agoniste dopaminergique (T0) et dure 1 heure. L'effet des produits est évalué statistiquement par une analyse de variance sur mesures répétées suivie d'un test de Dunnett pour comparaison des effets des différentes doses à l'effet du véhicule (eau distillée).

### ◆ Résultats

A titre d'exemple, le tableau suivant montre les effets du produit de l'exemple 2 administré par voie s.c.

| **Produit** | **Dose** mg/kg s.c. | **rotations contralatérales** moyenne ± e.s.m. (n) |
|---|---|---|
| Véhicule (eau distillée) | 0 | 53,8 ± 12,8 (8) |
| Apomorphine | 0,02 | 497,2 ± 91,4 * (11) |
| | 0,00063 | 157,4 ± 32,1 (5) |
| Exemple 2 | 0,0025 | 337,3 ± 48,3 * (6) |
| | 0,01 | 553,8 ± 138,3 * (5) |

| | | |
|---|---|---|
| ** p<0,05 versus véhicule (n) = nombre de rats-* *e.s.m. = erreur standard par rapport à la moyenne* | | |

Le produit de l'exemple 2 est actif dans ce test dès la dose de 0,0025 mg/kg.

### EXEMPLE 13 : Propriétés anxiolytiques - Test de vocalisations ultrasoniques chez le rat.

### ◆ Principe

Lorsqu'un rat est placé dans un environnement associé préalablement à une expérience désagréable (chocs électriques sur les pattes), son anxiété se traduit par l'émission de cris non audibles (ou vocalisations ultrasoniques). L'activité anxiolytique d'un produit se manifeste par une réduction de la durée de ces vocalisations.

### ◆ Appareillage

Des boîtes standard (Coulbourn Instruments), placées dans des caissons insonorisants et ventilés, sont équipées d'un sol constitué de barreaux métalliques électrifiables (générateur de chocs et scrambler Med Associates Inc) et d'un microphone situé au centre du plafond. Les ultrasons sont transformés dans une gamme audible (bat-detector Buitenbedrijf). Les signaux ainsi modifiés sont filtrés puis traités (logiciel RTS, Engineering Design). Les spectrogrammes obtenus sont enregistrés sur bandes DAT.

### ◆ Méthode

Des rats mâles de souche Wistar pesant 180-200 g à leur arrivée sont logés par cage de quatre avec libre accès à l'eau et à la nourriture, de cinq jours avant le début de l'étude jusqu'à la fin de celle-ci. La procédure employée se décompose en trois étapes successives séparées par 24h, appelées entraînement, sélection et test. Durant la séance d'entraînement, les animaux sont placés individuellement dans les boîtes et y reçoivent six chocs électriques (0,8 mA, 8 s) distribués aléatoirement sur une période de sept minutes. La sélection consiste à placer chaque animal dans une boîte pendant deux minutes pour y recevoir un seul choc, et à l'y replacer trente minutes plus tard pour une séance d'enregistrement des vocalisations ultrasoniques de dix minutes ; les animaux dont la durée des vocalisations est inférieure à 90 secondes sont écartés de la suite de l'expérience. La phase de test se déroule de manière similaire à l'étape de sélection, les produits ou le véhicule étant en outre administrés à la fin de la séance de deux minutes. L'effet des produits est évalué statistiquement par une analyse de variance sur mesures répétées suivie d'un test de Dunnett pour comparaison des effets des différentes doses à l'effet du véhicule (eau distillée).

### ◆ Résultats

A titre d'exemple, le tableau suivant montre les effets du produit de l'exemple 2 administré par voie s.c. sous un volume de 1 ml/kg.

| **Dose Exemple 2** mg/kg s.c. | **durée des vocalisations ultrasoniques (s)** moyenne ± e.s.m. (n) |
|---|---|
| 0 | 254,3 ± 42,1 (7) |
| 0,0025 | 274,0 ± 59,0 (5) |
| 0,04 | 28,0 ± 5,6 * (5) |
| 0,63 | 22,0 ± 3,2 * (5) |

| | |
|---|---|
| *e.s.m. : erreur standard à la moyenne - n : nombre de rats* ** p < 0,05 versus véhicule* | |

Aux doses de 0,04 et 0,63 mg/kg, ce produit entraîne une diminution importante de la durée des vocalisations, ce qui traduit son activité anxiolytique.

### EXEMPLE 14 : Composition pharmaceutique

Formule de préparation pour 1000 comprimés dosés à 10 mg :

| | |
|---|---|
| Composé de l'exemple 2 | 10 g |
| Hydroxypropylcellulose | 2 g |
| Amidon de blé | 10 g |
| Lactose | 100 g |
| Stéarate de magnésium | 3 g |
| Talc | 3 g |

## Revendications

1. Composés de formule I, de configuration relative trans : 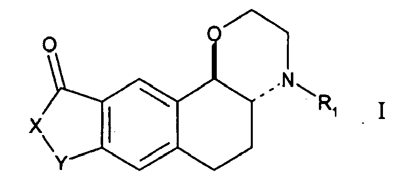 dans laquelle :
◆ X représente un atome d'oxygène ou un groupement NR₂,
◆ Y représente un groupement choisi parmi -CH₂-, -(CH₂)₂- et -CH=CH-,
◆ R₁ et R₂, identiques ou différents, représentent chacun un atome d'hydrogène ou un groupement choisi parmi alkyle C₁-C₆ linéaire ou ramifié, cycloalkyle C₃-C₈ et cycloalkylalkyle, dans lequel la partie alkyle est en C₁-C₆ et est linéaire ou ramifiée, et la partie cycloalkyle est en C₃-C₈,
sous forme racémique ou d'isomères optiques,
ainsi que leurs sels d'addition avec un acide pharmaceutiquement acceptable et leurs hydrates.

2. Composés de formule I selon la revendication 1, où R₁ représente un groupement alkyle.

3. Composés de formule I selon la revendication 1 ou 2, où X représente un groupement NR₂.

4. Composés de formule I selon l'une quelconque des revendications 1 à 3, où Y représente un groupement CH₂.

5. Composé de formule I selon la revendication 1 qui est choisi parmi :
- la (4a*RS* ,11b*RS*) 4-propyl-3,4,4a,5,6,8,9,11b-octahydroisoindolo[5,6-*h*][1,4]benzoxazin-10(2*H*)-one, ainsi que ses énantiomères et ses sels d'addition à un acide pharmaceutiquement acceptable;
- la (4a*R*,11b*R*) 4-propyl-2,3,4,4a,5,6,8,11b-octahydro-10*H*-furo[3',4':6,7] naphto[1,2-*b*][1,4] oxazin-10-one, ainsi que ses sels d'addition à un acide pharmaceutiquement acceptable;
- la (4aR,12bR) 4-propyl-3,4,4a,5,6,8,9,12b-octahydro-2*H*,11*H-*pyrano[4',3':6,7] naphto [1,2-*b*][1,4] oxazin-11-one, ainsi que ses sels d'addition à un acide pharmaceutiquement acceptable;
- la (4a*R*, 12b*R*) 4-propyl-2,3,4,4a,5,6,8,9,10,12b-décahydro-11*H* isoquino [6,7-h][1,4] benzoxazin-11-one, ainsi que ses sels d'addition à un acide pharmaceutiquement acceptable; et
- la (4a*R*, 12b*R*) 4-propyl-2,3,4,4a,5,6,10,12b-octahydro-11*H* isoquino [6,7-*h*][1,4] benzoxazin-11-one, ainsi que ses sels d'addition à un acide pharmaceutiquement acceptable.

6. Procédé de préparation des composés de formule I selon la revendication 1, à partir d'un composé de formule II, de configuration relative trans : 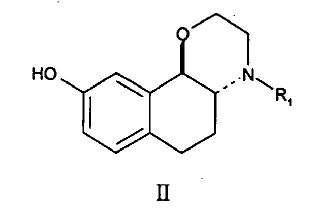 dans laquelle R₁ est tel que défini dans la formule I,
qui est mis en réaction avec de l'anhydride triflique en présence de pyridine pour conduire à un composé de formule III : 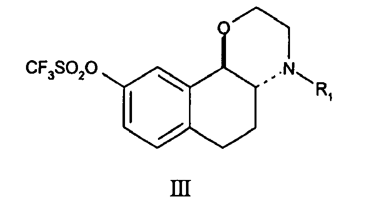 dans laquelle R₁ est tel que défini précédemment, qui est mis en réaction avec du cyanure de zinc et du tétrakis (triphénylphosphine)palladium (o) dans le diméthylformamide à chaud, pour conduire au composé de formule IV : 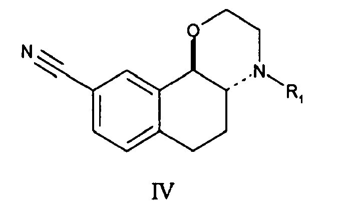 dans laquelle R₁ est tel que défini précédemment,
qui est traité par un mélange d'acide chlorhydrique et d'acide acétique au reflux, pour conduire à un composé de formule V: 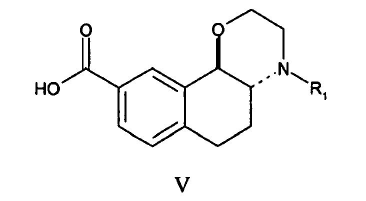 dans laquelle R₁ est tel que défini précédemment,
qui est ensuite transformé en composé de formule I, par des réactions classiques de la chimie organique.

7. Composition pharmaceutique contenant comme principe actif un composé selon l'une quelconque des revendications 1 à 5, en combinaison avec un ou plusieurs excipients ou véhicules inertes, non toxiques, pharmaceutiquement acceptables.

8. Utilisation des composés de formule I selon la revendication 1 pour la préparation de médicaments utiles dans le traitement des maladies du système nerveux central dans lesquelles le système dopaminergique est impliqué.

9. Utilisation des composés de formule I selon la revendication 1 pour la préparation de médicaments utiles dans la neuroprotection ou le traitement de la maladie de Parkinson, de l'hyperprolactinémie, de la dysfonction sexuelle, de la dépression, de l'anxiété, de la maladie d'Alzheimer ou d'autres maladies neurodégénératives comme les attaques cérébrales.

10. Composition pharmaceutique selon la revendication 7 utile pour le traitement des maladies du système nerveux central dans lesquelles le système dopaminergique est impliqué.

11. Composition pharmaceutique selon la revendication 7 utile pour la neuroprotection ou le traitement de la maladie de Parkinson, de l'hyperprolactinémie, de la dysfonction sexuelle, de la dépression, de l'anxiété, de la maladie d'Alzheimer ou d'autres maladies neurodégénératives comme les attaques cérébrales.

## Claims

1. Compounds of formula I, of *trans* relative configuration: 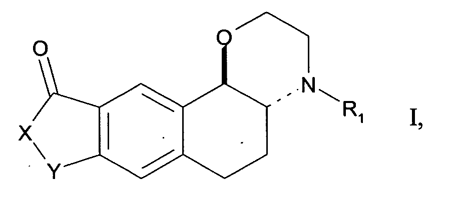 wherein:
◆ X represents an oxygen atom or an NR₂ group,
◆ Y represents a group selected from -CH₂-, -(CH₂)₂- and -CH=CH-,
◆ R₁ and R₂, which may be the same or different, each represents a hydrogen atom or a group selected from linear or branched C₁-C₆alkyl, C₃-C₈cycloalkyl, and cycloalkylalkyl wherein the alkyl moiety is C₁-C₆ and is linear or branched and the cycloalkyl moiety is C₃-C₈,
in racemic form or in the form of optical isomers,
and also addition salts thereof with a pharmaceutically acceptable acid, and hydrates thereof.

2. Compounds of formula I according to claim 1, wherein R₁ represents an alkyl group.

3. Compounds of formula I according to either claim 1 or claim 2, wherein X represents an NR₂ group.

4. Compounds of formula I according to any one of claims 1 to 3, wherein Y represents a CH₂ group.

5. Compound of formula I according to claim 1 which is selected from:
- (4a*RS*,11b*RS*)-4-propyl-3,4,4a,5,6,8,9, 11-b-octahydroisoindolo[5,6-*h*][1,4]benzoxazin-10(2*H*)-one, and also its enantiomers, and addition salts thereof with a pharmaceutically acceptable acid;
- (4a*R*,11b*R*)-4-propyl-2,3,4,4a,5,6,8,11b-octahydro-10*H*-furo[3',4':6,7]naphtho-[1,2-*b*][1,4]oxazin-10-one, and addition salts thereof with a pharmaceutically acceptable acid;
- (4a*R*,12b*R*)-4-propyl-3,4,4a,5,6,8,9,12b-octahydro-2H,11*H-*pyrano[4',3':6,7]naphtho-[1,2-*b*][1,4]oxazin-11-one, and addition salts thereof with a pharmaceutically acceptable acid;
- (4a*R*,12b*R*)-4-propyl-2,3,4,4a,5,6,8,9,10,12b-decahydro-11*H*-isoquino[6,7-*h*][1,4]-benzoxazin-11-one, and addition salts thereof with a pharmaceutically acceptable acid; and
- (4a*R*,12b*R*)-4-propyl-2,3,4,4a,5,6,10,12b-octahydro-11*H*-isoquino[6,7-*h*][1,4]-benzoxazin-11-one, and addition salts thereof with a pharmaceutically acceptable acid.

6. Process for the preparation of compounds of formula I according to claim 1, starting from a compound of formula II, of *trans* relative configuration: 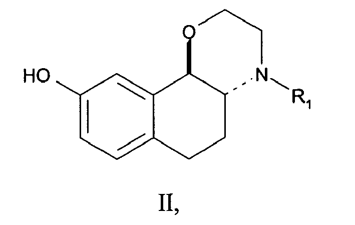 wherein R₁ is as defined for formula I,
which is reacted with triflic anhydride in the presence of pyridine to yield a compound of formula III: 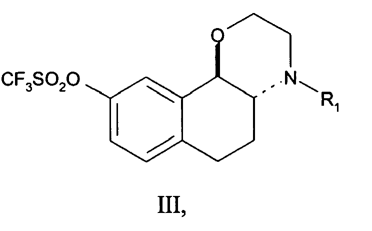 wherein R₁ is as defined hereinbefore,
which is reacted with zinc cyanide and tetrakis(triphenylphosphine)palladium(0) in dimethylformamide in the hot state to yield the compound of formula IV: 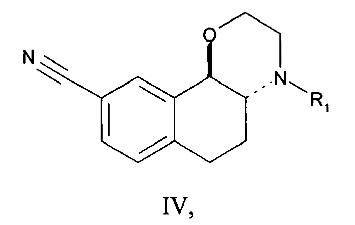 wherein R₁ is as defined hereinbefore,
which is treated with a mixture of hydrochloric acid and acetic acid under reflux to yield a compound of formula V: 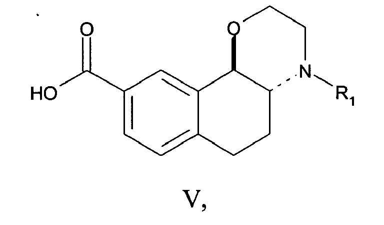 wherein R₁ is as defined hereinbefore,
which is then converted into a compound of formula I by conventional reactions of organic chemistry.

7. Pharmaceutical composition comprising as active ingredient a compound according to any one of claims 1 to 5 in combination with one or more inert, non-toxic, pharmaceutically acceptable excipients or carriers.

8. Use of compounds of formula I according to claim 1 in the preparation of medicaments for use in the treatment of disorders of the central nervous system that involve the dopaminergic system.

9. Use of compounds of formula I according to claim 1 in the preparation of medicaments for use in neuroprotection or treatment of Parkinson's disease, hyperprolactinaemia, sexual dysfunction, depression, anxiety, Alzheimer's disease or other neurodegenerative disorders such as cerebral attacks.

10. Pharmaceutical composition according to claim 7 for use in the treatment of disorders of the central nervous system that involve the dopaminergic system.

11. Pharmaceutical composition according to claim 7 for use in neuroprotection or treatment of Parkinson's disease, hyperprolactinaemia, sexual dysfunction, depression, anxiety, Alzheimer's disease or other neurodegenerative disorders such as cerebral attacks.

## Patentansprüche

1. Verbindungen der Formel I, in der relativen trans-Konfiguration: 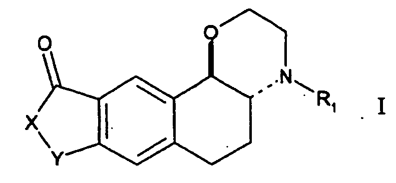 in der:
◆ X ein Sauerstoffatom oder eine Gruppe NR₂ bedeutet,
◆ Y eine Gruppe ausgewählt aus -CH₂-, -(CH₂)₂- und -CH=CH- bedeutet,
◆ R₁ und R₂, die identisch oder verschieden sind, jeweils ein Wasserstoffatom oder eine Gruppe ausgewählt aus geradkettigem oder verzweigtem C₁-C₆-Alkyl, C₃-C₈-Cycloalkyl und Cycloalkylalkyl, bei dem der Alkylteil C₁-C₆ und geradkettig oder verzweigt ist und der Cycloalkylteil C₃-C₈ ist, bedeuten,
in racemischer Form oder in Form der optischen Isomeren,
sowie deren Additionssalze mit einer pharmazeutisch annehmbaren Säure und ihre Hydrate.

2. Verbindungen der Formel I nach Anspruch 1, worin R₁ eine Alkylgruppe bedeutet.

3. Verbindungen der Formel I nach Anspruch 1 oder 2, worin X eine Gruppe NR₂ bedeutet.

4. Verbindungen der Formel I nach einem der Ansprüche 1 bis 3, worin Y eine CH₂-Gruppe bedeutet.

5. Verbindung der Formel I nach Anspruch 1, ausgewählt aus:
- (4a*RS*,11b*RS*)-4-Propyl-3,4,4a,5,6,8,9,11b-octahydroisoindol[5,6-*h*][1,4]benzoxazin-10(2*H*)-on sowie dessen Enantiomere und dessen Additionssalze mit einer pharmazeutisch annehmbaren Säure;
- (4a*R*,11b*R*)-4-Propyl-2,3,4,4a,5,6,8,11b-octahydro-10*H*-furo[3',4':6,7]naphtho[1,2-*b*]-[1,4]-oxazin-10-on sowie dessen Additionssalze mit einer pharmazeutisch annehmbaren Säure;
- (4a*R*,12b*R*)-4-Propyl-3,4,4a,5,6,8,9,12b-octahydro-2*H*,11*H*-pyrano[4',3':6,7]naphtho[1,2-*b*][1,4]-oxazin-11-on sowie dessen Additionssalze mit einer pharmazeutisch annehmbaren Säure;
- (4a*R*,12b*R*)-4-Propyl-2,3,4,4a,5,6,8,9,10,12b-decahydro-11*H*-isochino[6,7-*h*][1,4]-benz-oxazin-11-on sowie dessen Additionssalze mit einer pharmazeutisch annehmbaren Säure; und
- (4a*R*,12b*R*)-4-Propyl-2,3,4,4a,5,6,10,12b-octahydro-11*H*-isochino[6,7-*h*][1,4]-benzoxazin-11-on sowie dessen Additionssalze mit einer pharmazeutisch annehmbaren Säure.

6. Verfahren zur Herstellung der Verbindungen der Formel I nach Anspruch 1, ausgehend von einer Verbindung der Formel II, in der relativen trans-Konfiguration: 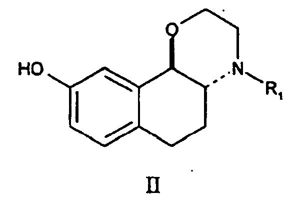 in der R₁ die bezüglich der Formel I angegebenen Bedeutungen besitzt,
welche in Gegenwart von Pyridin mit Trifluormethansulfonsäure (Triflinsäure) umgesetzt wird zur Bildung der Verbindung der Formel III: 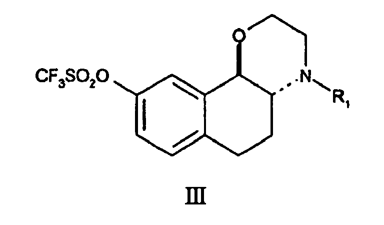 in der R₁ die oben angegebenen Bedeutungen besitzt,
welche mit Zinkcyanid und Tetrakis(triphenylphosphin)-palladium(O) in Dimethylformamid in der Wärme umgesetzt wird zur Bildung der Verbindung der Formel IV: 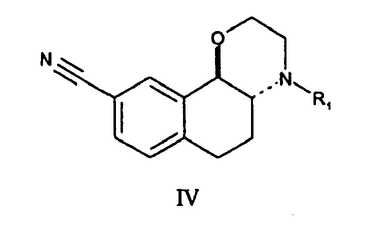 in der R₁ die oben angegebenen Bedeutungen besitzt, welche mit einer Mischung aus Chlorwasserstoffsäure und Essigsäure am Rückfluss umgesetzt wird zur Bildung der Verbindung der Formel V: 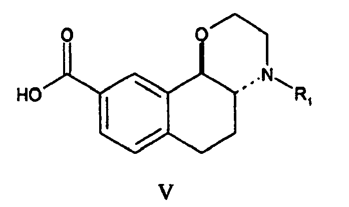 in der R₁ die oben angegebenen Bedeutungen besitzt,
welche anschließend mit Hilfe klassischer Reaktionen der organischen Chemie in die Verbindung der Formel I umgewandelt wird.

7. Pharmazeutische Zubereitung enthaltend als Wirkstoff eine Verbindung nach einem der Ansprüche 1 bis 5 in Kombination mit einem oder mehreren inerten, nichttoxischen, pharmazeutisch annehmbaren Hilfsstoffen oder Trägermaterialien.

8. Verwendung der Verbindungen der Formel I nach Anspruch 1 für die Herstellung von Arzneimitteln, die nützlich sind bei der Behandlung von Erkrankungen des Zentralnervensystems, bei denen das dopaminergische System betroffen ist.

9. Verwendung der Verbindungen der Formel I nach Anspruch 1 für die Herstellung von Arzneimitteln, die nützlich sind zur Neuroprotektion oder zur Behandlung der Parkinsonschen Krankheit, der Hyperprolactinämie, der sexuellen Dysfunktion, der Depression, der Angst, der Alzheimerschen Krankheit oder anderen neurodegenerativen Erkrankungen, wie Schlaganfällen.

10. Pharmazeutische Zubereitung nach Anspruch 7, nützlich zur Behandlung von Erkrankungen des Zentralnervensystems, bei denen das dopaminergische System betroffen ist.

11. Pharmazeutische Zubereitung nach Anspruch 7, nützlich zur Neuroprotektion oder zur Behandlung der Parkinsonschen Krankheit, der Hyperprolactinämie, der sexuellen Dysfunktion, der Depression, der Angst, der Alzheimerschen Krankheit oder anderen neurodegeneretativen Erkrankungen, wie Schlaganfällen.
